Europäisches Patentamt

European Patent Office

Office européen des brevets

(11) Publication number: **0 436 897 A2**

# EUROPEAN PATENT APPLICATION

(21) Application number: 90124871.6

(22) Date of filing: 20.12.90

(51) Int. Cl.⁵: **G01N 33/52**, //G01N33/70, G01N33/72, G01N33/92, C12Q1/60

(30) Priority: **12.01.90 US 468172**

(43) Date of publication of application:
**17.07.91 Bulletin 91/29**

(84) Designated Contracting States:
**DE FR GB IT**

(71) Applicant: **MILES INC.**
**1127 Myrtle Street**
**Elkhart Indiana 46514(US)**

(72) Inventor: **Barkes, Brian R.**
**50830 Lincroft Lane**
**Granger, Indiana 46530(US)**
Inventor: **Clements, Helen M.**
**52690 C.R. 21**
**Bristol, Indiana 46507(US)**
Inventor: **Magers, Thomas A.**
**63251 Mulberry Road**
**South Bend, Indiana 46614(US)**
Inventor: **Means, Margaret F.**
**1010 S 26th Street**
**South Bend, Indiana 46615(US)**
Inventor: **Skjold, A. Christopher**
**1602 Victoria Drive**
**Elkhart, Indiana 46514(US)**

(74) Representative: **Dänner, Klaus, Dr. et al**
**c/o Bayer AG Konzernverwaltung RP Patente**
**Konzern**
**W-5090 Leverkusen 1 Bayerwerk(DE)**

(54) Device and method of separating and assaying whole blood.

(57) A device and method of separating the cellular components of whole blood from plasma or serum and assaying the plasma or serum for a soluble constituent. The device includes a filter pad, that separates the cellular components of whole blood from the serum or plasma, in releasable contact with a test pad, that assays the serum or plasma for a particular soluble constituent. The filter pad, contaminated with the cellular components, is detachable from the plasma or serum-saturated test pad, thereby eliminating assay interference by the cellular components of whole blood. The method includes contacting the whole blood with a test device including a filter pad comprising a suitable carrier matrix optionally incorporating a lectin, a thrombin, or a mixture thereof, such that the cellular components of the whole blood are separated from the plasma or serum as the blood permeates through the filter pad. The essentially cell-free plasma or serum then saturates a test pad that is in releasable contact with the filter pad. After the plasma or serum saturates the test pad, the filter pad is detached from the test pad, and the exposed test pad is examined for a qualitative or quantitative response to a particular soluble constituent of the whole blood.

# DEVICE AND METHOD OF SEPARATING AND ASSAYING WHOLE BLOOD

## FIELD OF THE INVENTION

The present invention relates to a device and method of separating the cellular components of whole blood from the plasma or serum, and assaying the plasma or serum for a particular soluble constituent. More particularly, the present invention relates to an improved method of removing cellular components from whole blood by utilizing a filter pad comprising a suitable carrier matrix optionally incorporating a lectin, a thrombin or a combination thereof. The essentially cell-free plasma or serum, in an undiluted and unaltered form, then saturates a reagent-impregnated test pad that is in releasable contact with the filter pad. After the undiluted plasma or serum saturates the test pad, the filter pad, contaminated with the cellular components of the whole blood, is separated from the test pad and detached from the test device. The exposed test pad then is examined for a response to provide a prompt and accurate qualitative or quantitative assay for one or more soluble constituents of the plasma or serum.

## BACKGROUND OF THE INVENTION

Presently, numerous test devices are available to simply and rapidly analyze body fluids for the presence or absence of a particular soluble constituent. For example, tests are available to detect glucose, uric acid or protein in urine, or to detect glucose, triglycerides, potassium ion or cholesterol in blood. Historically, assays of a whole blood sample for a particular soluble constituent are the most difficult tests to design.

The cellular components of whole blood, and especially the red blood cells, are the primary interfering substances in assays for a soluble constituent of whole blood. Most simple blood tests are chromogenic, whereby a soluble constituent of the whole blood interacts with a particular reagent either to form a uniquely-colored complex or derivative as a qualitative indication of the presence or absence of the constituent, or to form a colored complex or derivative of variable color intensity as a quantitive indication of the presence of the constituent. The deep red color of the whole blood sample substantially interferes with these chromogenic tests, and therefore the highly-colored red blood cells usually are separated from the plasma or serum before the blood sample is assayed for a particular soluble constituent.

The presence of red blood cells also can interfere with various nonchromogenic blood assays, whereby the assay results are either inconsistent or, if consistent, are inaccurate. Furthermore, other cellular components, including the white blood cells, also can interfere in standard chromogenic blood assays. Therefore, to achieve a reliable assay for a soluble constituent of whole blood, it is essential to separate the serum or plasma from the cellular components of whole blood prior to analyzing the whole blood sample for a soluble component.

Conventionally, the plasma or serum is separated from the cellular material of whole blood by centrifugation. The cellular material collects at the bottom of the centrifuge tube and the supernatant plasma or serum is decanted. Accordingly, the interfering cellular components of whole blood are sufficiently removed such that a substantial background interference is avoided. However, the centrifuge method has the major disadvantages of requiring a relatively large blood sample, usually from about 0.1 ml to about 5 ml, and a long centrifuge time of approximately 5 to 10 minutes. Furthermore, the centrifuge method requires several manipulative steps. Consequently, laboratory technicians possibly can contact a potentially infectious blood sample, or laboratory equipment contaminated by the relatively large blood sample, and contract a disease.

Overall, the centrifuge method is most suited for large, automated laboratories that assay a multitude of blood samples, and for institutions, such as hospitals, that do not require assay results in a matter of minutes. However, many small laboratories and private medical offices do not have such a blood separator on site. Therefore, simple chromogenic tests cannot be performed quickly, safely and easily on site and the whole blood sample must be sent to an outside laboratory for efficient and safe separation and assay. As a result, the assay results are not available in minutes but in hours or days.

Accordingly, investigators have continually sought a device and method of quickly, safely and easily separating essentially all of the interfering cellular components of whole blood from the plasma or serum such that the identity and concentration of soluble constituents in the plasma or serum are not altered. Consequently, an assay for a particular constituent of the plasma or serum is trustworthy, accurate and free from interference by the cellular components of the whole blood. Investigators have provided several methods and devices for separating the interfering cellular components of whole blood from the plasma or serum. However, each method and device possessed at least one disadvantage that made the method or device inaccurate, cumbersome or impractical in assaying a whole blood sample for a particular soluble component.

Methods other than centrifugation have been used to separate the cellular components of a small whole blood sample from the serum or plasma. One of the simpler methods, as disclosed by Adams et al in U.S. Patent No. 3,092,465, uses a bibulous, or moisture absorbing, matrix that is impregnated with a chromogenic testing reagent and coated with a semipermeable barrier. The semipermeable barrier retains the cellular components of the whole blood sample and permits passage of the smaller molecules and ions to contact the chromogenic testing reagent impregnated in the bibulous matrix. In the case of a positive test, the essentially colorless plasma or serum interacts with the chromogenic testing reagent to produce a color in the bibulous matrix. The color is observed by water rinsing or wiping away the cellular material retained on the semipermeable barrier. However, the rinsing or wiping technique is cumbersome and laborious, and assay interference is possible if the red blood cells are not completely wiped or rinsed from the semipermeable barrier. In addition, the possibility of technician contact with the potentially infectious blood sample is high.

Fetter in U.S. Patent Nos. 3,552,925 and 3,552,928 disclosed another method and device to assay small whole blood samples for soluble constituents. Fetter described a test device having a bibulous matrix impregnated with a non-volatile inorganic salt or an amino acid at a first region on the matrix and impregnated with a test reagent at an adjacent second region of the matrix. A whole blood sample is introduced onto the bibulous matrix such that the whole blood first contacts the first region of the bibulous matrix including the inorganic salt or amino acid. The salt or amino acid precipitates the cellular components from the blood, and the plasma or serum then migrates to the test reagent-impregnated second region of the bibulous matrix for chromogenic interaction with the test reagent. The salts or amino acids used in this process effectively separate the red blood cells from the whole blood sample, but also introduce contaminating ions or molecules into the plasma or serum and precipitate a portion of the soluble plasma or serum constituents. Therefore a quantitative assay for a soluble constituent of the plasma or serum is unreliable. The Fetter method does eliminate a distinct manipulative step in the separation of the cellular components of whole blood from the plasma or serum. However, the method suffers from the disadvantage that the plasma or serum may no longer contain the true concentration of the soluble constituents of interest.

Another prior art method of separating the cellular components of whole blood from the plasma or serum is disclosed by Vogel et al, in U.S. Patent No. 4,477,575, describing a process and a composition for separating plasma or serum from whole blood using a layer of glass fibers having a defined average diameter and density. In addition to the defined glass fiber parameters, the amount of plasma or serum that can be separated is limited to at most 50%, and preferably less than 30%, of the absorption volume of the glass fibers. Otherwise, whole blood, containing approximately 50% filterable cellular material, effectively clogs the glass fiber layer. Therefore, the method requires a high ratio of hydrophobic glass fibers to whole blood volume.

Furthermore, in many prior art methods, the whole blood is diluted before assaying for a soluble plasma or serum constituent. The dilution of whole blood is burdensome because an extra manipulative step is required, and dilution introduces the possibility of assay error because of an incorrect dilution of the blood sample. The possibility of technician contact with the potentially infectious blood sample also is increased. For example, German Patent No. 34 41 149 disclosed a method of separating plasma or serum from whole blood by passing the whole blood through a lectin-impregnated matrix that is repeatedly rinsed with a diluent to dilute the plasma or serum before the assay is performed. However, the possibility of imprecise dilution can result in an inaccurate assay for the plasma or serum constituent of interest.

In developing a method and device for separating and assaying small whole blood samples, a primary consideration is the degree of sophistication of the technician performing the assay. Often it is desirable to have relatively untrained personnel perform routine assays and obtain accurate quantitative results. Therefore, it is important that the assay method include a minimum of manipulative steps, be free of possible interferences or contamination, minimize or eliminate the possibility of the laboratory personnel from physically contacting the blood sample, and provide for easy measurement. For instance, among the several possible manipulative steps, the dilution of the whole blood, or the plasma or serum, prior to the actual assay introduces the most probable step for assay error or personal contact with the blood sample. Another common manipulative error is the incomplete wiping or rinsing of the cellular components of whole blood from the surface of a device that utilizes a cell-impermeable membrane to separate the cellular components from the plasma or serum of whole blood.

Therefore, a need exists for a method and device to efficiently separate and accurately assay small

3

volumes of whole blood. The method preferably avoids a distinct manipulative step to separate the cellular components from the plasma or serum prior to the assay. Furthermore, in order to avoid dilution errors, the method preferably allows the assay of undiluted plasma or serum. In addition, the device preferably avoids the use of complicated and expensive multilayered test strips. It also is desirable to have a blood separation and blood assay method that shields the technician from contact with the blood sample; that avoids the time delays of the present methods; and that yields accurate and reproducible results.

The ideal method includes withdrawing a whole blood sample in "noninvasive" amounts, such as a pin prick drop, and immediately depositing the undiluted whole blood sample on a single separating-analyzing device, whereby the cellular components are separated from the undiluted plasma or serum and the presence or concentration of a plasma or serum constituent is determined within minutes. Alternatively, the separating-analyzing device can contact a fresh puncture wound and thereby withdraw a blood sample from the wound for analysis. Such a separation and assay method and device would allow medical personnel to carry out whole blood analyses on a more routine and more confident basis.

Consequently, investigators have attempted to develop test devices that include an element to separate, collect and retain the cellular components of whole blood. In addition, some test devices were developed wherein the cell-separating element can be physically disconnected from the test device and discarded before assaying the plasma or serum for a particular constituent. For example, the previously-mentioned Vogel et al U.S. Patent No. 4,477,575 disclosed a device wherein a glass fiber cell-separating layer is disposed over a reaction layer, such that the separating layer can be removed from the reaction layer. The reaction layer then can be examined for a response to a particular plasma or serum constituent. However, the glass fiber separating layer of the Vogel device requires the disadvantageous high ratio of glass fiber to blood described above. Consequently, a specific volume of blood must be pipetted onto the test device, thereby adding a time-consuming manipulative step that can result in operator error and erroneous assays. Such a manipulative step also can lead to reduced operator safety because of potential physical contact between the operator and the blood sample.

Similarly, Rothe et al, in U.S. Patent No. 4,223,089, described a dry phase test strip to assay for ammonia wherein plasma or serum is applied to a porous membrane over a reaction layer. Upon contacting the plasma or serum, the reagents in the reaction layer convert the urea in the plasma or serum to ammonia. The gaseous ammonia diffuses through a porous spacer below the reaction layer to permeate an indicator layer that changes color in response to the amount of ammonia generated. This device allows the top reaction layer to be physically disconnected from the indicator layer to permit examination of the indicator layer for a response to ammonia.

Rothe et al, in U.S. Patent No. 4,604,264, also disclosed a variation of the features of Rothe et al U.S. Patent No. 4,223,089 and of Vogel et al U.S. Patent No. 4,477,575 in a hinged assay device. This particular device of Rothe et al includes a separating layer consisting of a glass fiber fleece, wherein the whole blood is applied near the end of the separating layer distant from the hinge area of the device. As the blood permeates through the separating layer, the cellular components are separated from the serum or plasma. The serum or plasma then migrates towards the hinge of the device to an area of the separating layer underneath a reaction/indicator layer that is disposed above the separating layer and secured to the separating layer by a hinge. By pressing down on the reaction/indicator layer, contact between the the lower face of reaction/indicator layer and the separating layer allows the reaction/indicator layer to absorb the serum or plasma for reaction with reagents in the reaction/indicator layer. Assay detection, such as a color change, is achieved by observation through a transparent film disposed on the upper face of the reaction/indicator layer.

Kennedy et al, in application PCT/US86/02192, disclosed a disposable dry phase test stick having a reactive area covered by a semipermeable membrane. The semipermeable membrane separates cellular and particulate matter from whole blood and allows the plasma or serum to contact the reactive area. The semipermeable membrane can be detached from the reactive area to remove cellular components and particulate matter from the device and to expose the reactive area for examination of a response to a particular analyte. The semipermeable membrane is a polytetrafluoroethylene material made hydrophilic with a surfactant or soap-like wetting agent. However, the surfactant or soap-like wetting agent can remove particular noncellular components from the plasma or serum, such as potassium ions, or can contaminate the plasma or serum.

Another prior art patent directed to separating the cellular components of whole blood from the plasma or serum is Rapkin et al U.S. Patent No. 4,678,757, wherein a carbohydrate-treated permeable carrier is used to separate the cellular components of whole blood from the plasma or serum. The plasma or serum of the whole blood then contacts a reagent-treated permeable carrier for assay of a particular blood constituent. In this device, the cellular components collect and are retained at the bottom edge of the

4

carbohydrate-treated carrier, and the plasma or serum permeates through the carbohydrate-treated carrier to contact the reagents necessary for the assay. Assay results are deter-mined by observation through a transparent material covering the permeable layers. In the Rapkin et al. device, the layer collecting and retaining the cellular components is not physically disconnected from the device before examining the device for a response to a particular blood constituent.

In addition, Terminiello et al, in U.S. Patent No. 4,774,192, disclosed a porous membrane having a porosity gradient such that the cellular components of whole blood are retained in an area of the membrane having a low porosity. The serum or plasma can flow through the area of low porosity to contact assay reagent components incorporated into an area of the membrane having a high porosity. Furthermore, G. Rayman and J.L. Day, in the publication "New Device to Improve the Accuracy of Bedside Blood Glucose Tests", The Lancet, Nov. 12, 1988, pp. 1107-1109, described a disposable test strip for glucose wherein the surface of the strip is wiped to clear the cellular components of the whole blood from the strip. Neither device incorporates an element to retain the cellular components of the whole blood that then is physically disconnected from the assay element of the device. In the Terminiello et al device, higher molecular weight soluble plasma components, such as cholesterol, may not completely permeate through the low porosity area of the membrane; and in the Rayman and Day device the cellular components may not be completely wiped from the surface of the strip. Therefore, in each device, inaccurate and unreliable assays may result.

Therefore, because of the disadvantages present in the above-cited prior art methods and test devices, it is apparent that a simple and effective method of separating the cellular components of whole blood to provide essentially cell-free, unaltered and undiluted plasma or serum is needed. Accordingly, the method and device of the present invention allows the safe, accurate and economical assay of a whole blood, or other biological fluid, sample for a particular soluble component by utilizing a filter pad in releasable contact with a reagent-impregnated test pad to achieve essentially total separation of the cellular components of whole blood from the plasma or serum. The device allows the assay of whole blood without resorting to lengthy and expensive wet phase assays and without the need of complicated and costly multilayered test strips. In addition, the cell-free plasma or serum saturating the test pad is unaltered and undiluted, thereby allowing a more accurate and trustworthy assay for a soluble constituent. The method and device of the present invention also eliminate the disadvantages of hemocrit sensitivity, technique sensitivity due to wiping or rinsing the cellular components from the test device and disposal of the cellular components.

Furthermore, in accordance with the method and device of the present invention, after the whole blood sample has saturated the filter pad and the test pad, the filter pad retaining the cellular components of the blood is physically disconnected from the test device by peeling, tearing or snapping the filter pad from the device, and thereby exposing the test pad of the device. The test pad, saturated with undiluted and unaltered plasma or serum, then is examined for a response to a particular plasma or serum constituent by standard dry phase chemistry test strip procedures.

In accordance with another important feature of the present invention, the device essentially precludes contact between the technician and the blood sample. The blood sample is absorbed into the filter pad in such a manner that excess blood sample does not remain on an outside surface of the device. In addition, the cellular components do not have to be wiped or rinsed from the device before examination of the device for a response. Consequently, the device essentially eliminates the possibility of contact between the technician and a potentially infectious blood sample.

As a result, the assay of plasma or serum for a particular soluble constituent is accurate and reliable because the interferences attributed to the highly-colored cellular components are essentially eliminated. The prior art methods and devices rely either upon wiping the cellular components from the surface of the analyte detection device or upon immobilizing the cellular components, physically or chemically, in an area of the analyte detection device distant from the actual assay area, usually in a complicated multilayered test strip. As will be demonstrated more fully hereinafter, the device of the present invention provides an accurate and economical method of first separating the cellular components of whole blood from the plasma or serum. Then, the element retaining the interfering cellular components is completely detached from the test device to permit examination of the exposed assay area of the device for a response to a particular component of the plasma or serum. The assay is detected by conventional detection techniques, either visual or instrumental, without the additional opacifying or transparent layers often included in a multilayered device to help eliminate the interfering effects of the cellular components of the whole blood. Furthermore, the serum or plasma is distributed evenly throughout the entire test pad, therefore the assay response is homogeneous throughout the test area of the device. Consequently, a simple and accurate assay detection can be achieved at any position of the test area. In addition, both the detached filtering element and the test area of the device are discarded after use, thereby precluding contact between the technician and the blood sample.

## SUMMARY OF THE INVENTION

In brief, the present invention is directed to a device and method of separating the cellular components from whole blood and assaying the undiluted and unaltered serum or plasma for a particular soluble constituent, or constituents, without additional manipulative steps. It has been found that separating the cellular components of whole blood from the plasma or serum by the method and device of the present invention provides an essentially cell-free, undiluted and unaltered serum or plasma sample for immediate assay of a particular soluble constituent. The separation method and device do not introduce contaminants into the serum or plasma, and do not alter the compositional makeup of the plasma or serum. Furthermore, the method and test device of the present invention also can be used to assay other biological fluids having cellular or particulate matter that interfere in an assay for a particular soluble constituent. In addition, the device includes an element to separate the cellular components of the whole blood from the plasma or serum that is detachable from the device, thereby both preventing the inadvertent contamination of the serum or plasma by the cellular components and facilitating the assay of the serum or plasma for a particular constituent. The method and device also essentially precludes technician contact with a potentially infectious blood or other biological sample.

In accordance with an important feature of the present invention, the device includes a filter pad, that separates the cellular components of whole blood from the serum or plasma, in releasable contact with a test pad that assays the serum or plasma for a particular soluble constituent. The filter pad comprises a suitable carrier matrix. In addition, the carrier matrix can be impregnated with a separating reagent to facilitate the separation of the cellular blood components from the serum or plasma. In accordance with the present invention, the filter pad, contaminated with the cellular components of the whole blood sample, is detachable from the plasma or serum saturated test pad, thereby precluding the cellular components from interfering with the assay for a soluble constituent of the blood or plasma. The serum or plasma, in an undiluted and unaltered form, is assayed for a particular soluble constituent by the specific indicator reagent composition incorporated into the test pad. Detaching the filter pad from the device allows examination of the exposed test pad for a response, such as a color change, to a particular soluble constituent of the whole blood. In addition, the present invention not only eliminates technique dependence of the assay, such as eliminating the manipulative step of wiping or rinsing the cellular components from a multilayered test device, but also assures that the proper volume of plasma or serum contacts the test pad of the device. Furthermore, technician contact with the potentially infectious blood or other biological sample is essentially precluded.

The method includes first contacting a whole blood sample with a test device comprising a filter pad, including a suitable carrier matrix and optionally impregnated with a separating reagent, and a test pad, including a suitable substrate material incorporating an indicator reagent composition. As used here, and hereinafter, the expression "separating reagent" is defined as a chemical or mixture of chemicals that effects a separation of the cellular components of whole blood from the remaining soluble blood constituents. Likewise, as used here and hereinafter, the expression "indicator reagent composition" is defined as a chemical or mixture of chemicals causing an observable or detectable interaction upon contact with the substance being detected. The whole blood sample initially contacts only the filter pad of the test device and permeates through the filter pad wherein the red blood cells and other cellular and particulate components of the whole blood sample are separated from the plasma or serum through the action of the carrier matrix and, if present, the separating reagent. The undiluted and unaltered plasma or serum continues to permeate through the filter pad to contact a test pad that is in releasable contact with the filter pad. After the plasma or serum saturates the test pad, the filter pad is physically disconnected, or detached, from the test pad, such as by peeling, tearing or snapping the filter pad from the device, and discarded.

The assay of interest is performed by the test pad of the device. The undiluted plasma or serum interacts with the indicator reagent composition previously incorporated into the test pad to produce a detectable change in the test pad, such as a color change, to show the presence or absence of a particular soluble constituent or to permit a quantitative determination of the particular soluble constituent. After detaching the filter pad from the device, the exposed test pad is examined, either visually or instrumentally, for a qualitative or quantitative response to the particular soluble constituent of interest. After examination for a response, the test pad of the device also is discarded. Consequently, the assay for the particular soluble constituent of interest is achieved by using a simple and inexpensive test device that effectively separates the interfering cellular and particulate components from the test sample without the need of multiple screening and filtering layers and without manipulative steps, such as wiping or rinsing, that can lead to technician error or contact with the potentially infectious blood sample.

Therefore, the present invention is directed to a method and device for rapidly and effectively separating the cellular components from the plasma or serum of undiluted whole blood and assaying the plasma or serum for a particular soluble constituent. More particularly, and in accordance with an important feature of the present invention, one or more, untreated or chemically-treated, filter pads are arranged to separate the cellular components from whole blood and to allow the plasma or serum to pass onto one or more test pads that are in releasable contact with the filter pad, or pads. After the test pad is saturated with plasma or serum, the filter pad is separated from the test pad, and the exposed test pad is examined for a qualtitative or quantitative response to soluble serum or plasma constituents. Accordingly, assay interferences attributed to the cellular components of whole blood are eliminated, thereby achieving a more accurate and more reliable serum or plasma assay.

In a preferred embodiment, the whole blood contacts a test device comprising a filter pad including a suitable carrier matrix impregnated with a thrombin. To achieve the full advantage of the present invention, the whole blood contacts a test device comprising a filter pad including a suitable carrier matrix impregnated with a lectin. The thrombin or lectin-impregnated filter pad facilitates separation of the cellular components of the whole blood from the plasma or serum and does not add contaminating ions or molecules to, or remove soluble constituents from, the serum or plasma. The amount of lectin included in the filter pad is in the range of from about 55 to about 40,000 units per $cm^3$ of carrier matrix material comprising the filter pad, wherein each "unit" is defined as the amount of lectin necessary to agglutinate a two percent solution of red blood cells within one hour of incubation at 25°C. The amount of thrombin included in the filter pad is in the range of from about 90 to about 900 NIH (National Institute of Health) units per $cm^3$ of carrier matrix material comprising the filter pad. The undiluted serum or plasma passes through the filter pad of the device, essentially unimpeded, to saturate the test pad of the device and the assay of interest is performed. The test pad of the device is in releasable contact with the filter pad and comprises an indicator reagent composition homogeneously incorporated into a suitable substrate material. In accordance with an important feature of the present invention, the particular plasma or serum constituent of interest is detected or measured without diluting the plasma or serum, and without any wiping or rinsing of the test device or performing any other manipulative steps.

Therefore, it is an object of the present invention to provide a method and device to quickly and effectively separate the cellular and other particulate components from the plasma or serum of small whole blood samples or other biological samples.

It is also an object of the present invention to provide a method and device for the rapid, easy and effective separation of undiluted and unaltered plasma or serum from the cellular components of whole blood.

Another object of the present invention is to provide a method and device to separate the cellular components of whole blood from the plasma or serum, and then to assay the plasma or serum for a particular soluble constituent without additional manipulative steps.

Another object of the present invention is to provide a method and device to eliminate interferences attributed to the cellular components of whole blood in assays of undiluted and unaltered plasma or serum for soluble constituents.

Another object of the present invention is to provide a method and device to eliminate technician contact with a potentially infectious whole blood or other biological sample.

Another object of the present invention is to provide a method of separating the cellular components from whole blood by utilizing an agglutinizing compound, a coagulating compound, or a combination thereof.

Another object of the present invention is to provide a method of separating the cellular components from whole blood by utilizing a lectin, a thrombin, or a combination thereof.

Another object of the present invention is to provide a new and improved test device for the qualitative or quantitative analysis of a small sample volume of whole blood.

Another object of the present invention is to provide a new and improved test device to qualitatively or quantitatively assay whole blood that essentially eliminates assay interferences attributed to the cellular components of the whole blood sample.

Another object of the present invention is to provide a test device for assaying an undiluted whole blood sample for a particular soluble constituent comprising a filter pad to remove the cellular components of the whole blood in releasable contact with a test pad incorporating an indicator reagent composition, to assay the plasma or serum for the particular soluble constituent.

Another object of the present invention is to provide a dry phase test strip device including a test pad, comprising a substrate materialcapable of homogeneously incorporating an indicator reagent composition that interacts with the constituent of interest in the serum or plasma, in releasable contact with a filter pad, comprising a suitable carrier matrix capable of homogeneously incorporating a separating reagent composition that interacts with the cellular components of whole blood.

Another object of the present invention is to provide a new and improved test device and method of manufacturing the test device for sensing the presence of a chemical compound in whole blood, wherein the test device comprises a filter pad in releasable contact with a test pad, such that a whole blood sample contacting the filter pad first is separated into cellular components and into undiluted plasma or serum with the cellular components remaining in the filter pad and the undiluted plasma or serum permeating through the filter pad; then, after the undiluted plasma or serum sufficiently contacts a test pad to produce a detectable or measurable response, the filter pad is detached from the test device to allow either visual or instrumental examination of the exposed test pad for a response to the chemical compound of interest.

## BRIEF DESCRIPTION OF THE DRAWINGS

The above and other objects, advantages and novel features of the present invention will become apparent from the following detailed description of the present invention taken in conjunction with the drawing wherein:

FIG. 1 is a partial side view of a test device for separating the cellular components of whole blood or other biological samples from the plasma or serum and for assaying the plasma or serum for a particular soluble constituent;

FIG. 2 is an end view of the test device of FIG. 1 taken in the direction of arrows 2-2 of FIG. 1 showing the sample port for introducing the whole blood or other biological sample to the test device;

FIGS. 3 and 4 are a side view and a top view, respectively, of the test device of FIG. 1 before the filter pad is positioned in releasable contact with the test pad;

FIGS. 5 and 6 are views similar to FIG. 1 showing alternate embodiments of the test device of the present invention;

FIGS. 7 and 8 are views similar to FIG. 6 and FIG. 1 showing alternate embodiments of the test device of the present invention useful for assaying more than one soluble plasma or serum constituent;

FIG. 9 is an expanded side view of the assembly elements of a preferred embodiment of a test device for separating the cellular components of whole blood from the plasma or serum and for assaying the plasma or serum for a particular soluble constituent;

FIG. 10a-10c illustrate the use of a test device of the present invention in the assay of a whole blood sample for a particular soluble constituent; and

FIG. 11 is a plot of the Kubelka-Munk function (K/S) vs. the cholesterol concentration of standardized test samples assayed for cholesterol with test devices of the present invention.

## DETAILED DESCRIPTION OF THE INVENTION

In accordance with the method of the present invention, the cellular components of a whole blood sample first are effectively separated from the plasma or serum; then, the undiluted and unaltered plasma or serum is assayed for a particular soluble constituent, or constituents, without any further manipulative steps. According to the method and device of the present invention, a small blood sample, usually a pin prick amount, is sufficient to achieve separation of the cellular components and to assay the serum or plasma, as opposed to the large milliliter size blood samples required in the centrifuge method of separation. Furthermore, the method and device of the present invention eliminates the manipulative steps that can cause technician contact with the potentially infectious whole blood sample.

Surprisingly and unexpectedly, the test device of the present invention essentially completely separates the highly-colored and interfering red blood cells from the plasma or serum, and then assays the plasma or serum for a particular soluble constituent, within minutes, without the additional time-consuming and potentially unhealthful manipulative steps of either centrifugation, decantation or plasma or serum dilution. The method of the present invention provides rapid and reliable whole blood assays on undiluted, unaltered, and noncontaminated plasma or serum samples with a simple and inexpensive test strip device. Overall, the method and device of the present invention are ideally suited for routine blood assays at home, in small laboratoriesor private medical offices, wherein the number of assays usually is relatively low, but accurate results are nevertheless required in a short time period.

As will become apparent from the following detailed description of the invention, the method and device of the present invention are especially suited for blood assays utilizing chromogenic responses to determine the presence or concentration of the various soluble constituents of whole blood. Therefore, it is of primary importance to remove the highly-colored red blood cells from the whole blood sample in order to achieve an accurate and reliable detection and measurement of the chromogenic response. Furthermore, any method or device for separating the cellular components of whole blood from the plasma or serum should quickly and efficiently achieve cell separation; remove only the cellular components and not the soluble plasma or serum constituents; avoid contamination of the plasma or serum with interfering, soluble ions or molecules; and minimize or eliminate hemolysis, wherein the red blood cells rupture and release their highly-colored components to the plasma or serum. Due to the potentially infectious nature of the blood or other biological sample, the method and device also should minimize, or eliminate, human contact with the test sample.

In accordance with an important feature of the present invention, it has been found that the cellular components of whole blood are effectively separated from the essentially-colorless plasma or serum by allowing the whole blood sample to contact and permeate through a filter pad comprising a suitable carrier matrix that optionally has been impregnated with, or otherwise includes, a suitable separating reagent. The separating reagent assists the carrier matrix in the removal of the highly-colored cellular components of the whole blood sample to yield plasma or serum that is amenable to a simple and accurate determination of its soluble components.

In accordance with another important feature of the present invention, the filter pad of the test device is in releasable contact with the test pad of the test device. As the whole blood permeates through the filter pad, the cellular components are separated from the whole blood sample and collected and retained in the filter pad. The unaltered serum or plasma then advances to, and saturates, a test pad that is in releasable contact with the filter pad. The test pad comprises a suitable substrate material incorporating an indicator reagent composition that interacts with the plasma or serum constituent of interest to give a detectable or measurable response. After the test pad is saturated by the plasma or serum, the cell-contaminated filter pad is separated from the test pad and detached from the test device, thereby allowing either a visual or an instrumental examination of the exposed test pad for a response, such as a chromogenic response, to a particular plasma or serum constituent.

Generally, the filter pad of the present invention comprises a suitable carrier matrix capable of filtering the cellular components from a whole blood sample. The carrier matrix is normally a hydrophilic matrix capable of separatingthe cellular components of whole blood from the plasma or serum. Preferably, the carrier matrix also is capable of incorporating a separating agent to facilitate removal of the cellular components from the whole blood. In addition to collecting and retaining the separated cellular components, the carrier matrix, either untreated or treated with a separating agent, should permit the plasma or serum to permeate through the carrier matrix essentially unimpeded to contact the test pad.

The carrier matrix also should permit the whole blood sample to permeate through the filter pad at a sufficient rate to allow adequate time for efficient red blood cell separation, yet rapidly enough to obtain blood assays relatively quickly. In addition, the carrier matrix should not promote hemolysis, contaminate the serum or plasma by serum or plasma-extraction of components of the carrier matrix, remove serum or plasma constituents by chemical or physical interactions, or appreciably alter the undiluted plasma or serum in a way to make the subsequent blood assays inconclusive, inaccurate or doubtful.

Therefore, the filter pad of the present invention normally comprises a hydrophilic carrier matrix, possessing the above-mentioned characteristics, that allows the blood to move, in response to capillary forces, through the carrier matrix. The cellular components are separated from the plasma or serum and retained by the carrier matrix. The essentially unaltered serum or plasma then continues advancing through the carrier matrix to contact and saturate a test pad that is in releasable contact with the filter pad.

The carrier matrix can be any hydrophilic material that allows only the essentially cell-free plasma or serum to pass through the filter pad to contact the test pad for analysis of a particular soluble substituent. Suitable hydrophilic carrier matrices include bibulous and nonbibulous, fibrous and nonfibrous matrices, like hydrophilic inorganic powders, such as silica gel, alumina, diatomaceous earth and the like; sponge materials; argillaceous substances; cloth; hydrophilic natural polymeric materials, particularly cellulosic material, like cellulosic beads, and especially fiber-containing papers such as filter paper or chromatographic paper; and synthetic or modified naturally occurring polymers, such as cellulose acetate, polyvinyl chloride, polyacrylamide, polyacrylates, polyurethanes, crosslinked dextran, agarose, and other such crosslinked and noncrosslinked water-insoluble hydrophilic polymers. Similarly, other suitable carrier matrices include fibrous and nonfibrous matrices like a glass fiber matrix; and synthetic polymers, like polypropylene, polyethylene, nylon, polyvinylidene fluoride and polysulfones.

The carrier matrix can have a pore size of between about 0.1 u (microns) and about 50 u, and preferably between about 0.3 u and about 10 u, to achieve efficient separation of the cellular components and to permit the serum or plasma to advance through the filter pad. To achieve the full advantage of the present invention, the carrier matrix has a pore size ranging from about 0.5 u to about 8 u.

The filter pad of the test device can include more than one carrier matrix, and the carrier matrices can have different physical characteristics and can be of different chemical compositions or a mixture of chemical compositions. The carrier matrix, or matrices, of the filter pad also can vary in regards to smoothness and roughness combined with hardness and softness. However, in every instance, the carrier matrix, or matrices, of the filter pad separates and collects the cellular components of whole blood, and allows the plasma or serum to pass through the filter pad to the test pad unaltered and essentially unimpeded. Therefore, regardless of the exact composition of the carrier matrix or matrices, the primary considerations are separation, collection and retention of the cellular components of whole blood and transmittal of substantially unaltered and undiluted plasma or serum.

To achieve the full advantage of the present invention, the carrier matrix comprises a cellulosic material, such as paper, and preferably filter paper; or a glass fiber matrix. Both filter paper and a glass fiber matrix possess the properties required of a suitable carrier matrix of the present invention, plus the advantages of abundant supply, favorable economics, and a variety of suitable grades. Furthermore, filter paper and glass fiber matrices are capable of homogeneously incorporating a separating reagent in the filter pad, and of effectively separating the cellular components from whole blood.

As known to those skilled in the art, filter paper and glass fiber matrices are available in a variety of thicknesses and porosities. The thickness and porosity of the carrier matrix in turn influence the effectiveness of the separation process. The thickness and porosity of the carrier matrix are directly related to the ability of the carrier matrix to separate the cellular components from plasma or serum and to the time required for the whole blood sample to permeate through the carrier matrix to separate the cellular components from the whole blood sample. Therefore, if a carrier matrix of high porosity is used, the thickness of the carrier matrix should be sufficient to allow a minimum effective contact time between the whole blood and the filter pad in order to achieve an effective separation of the cellular components of the whole blood. Conversely, if a carrier matrix of low porosity is utilized, a relatively thin layer of carrier matrix can be employed. The proper balance between carrier matrix porosity and thickness, and a judicious selection of the type and concentration of separating reagent impregnated into the carrier matrix, is well within the experimental techniques used by those skilled in the art of preparing the test devices described in the present specification.

A carrier matrix of sufficient thickness and of sufficiently low porosity can separate the cellular components from the plasma or serum without incorporating a separating reagent into the carrier matrix. For example, the effective separation of the cellular components from a sample of whole blood has been observed as the blood permeates through an untreated carrier matrix having sufficient thickness and sufficiently low porosity. However, the separation of the cellular components of whole blood from plasma or serum is not observed if the carrier matrix is too thin or too porous. Therefore, if a thin or porous carrier matrix is impregnated with a suitable separating reagent, the cellular components are effectively removed and fixed in the carrier matrix as the blood sample permeates through the carrier matrix, and the plasma or serum advances through the carrier matrix to contact the test pad of the test device.

It has been found that an untreated carrier matrix having a thickness of at least about 1 mm (millimeters), and preferably of at least about 1.5 mm, effectively separates the cellular components from a whole blood sample. However, if a separating reagent is incorporated into the carrier matrix to assist removal of the cellular components from the serum or plasma, the carrier matrix can have a thickness of at least about 0.2 mm, and preferably at least about 0.3 mm. To achieve the full advantage of the present invention, the filter pad comprises a carrier matrix in the form of a pad, having dimensions of, for example, approximately 0.25 cm. (centimeters) by 1.0 cm to approximately 0.5 cm by 2 cm, and a thickness of approximately 0.2 mm to approximately 2 mm. A filter pad of such dimensions has sufficient length, width and thickness for effective cell removal within a reasonably short time after the blood contacts one end, or the top, of the filter pad.

If a test device of the present invention has a filter pad of the above-defined dimensions, a pin-prick amount of blood, such as about 0.02 ml (milliliters), usually is a sufficient amount of sample to provide a fast and accurate assay for a particular soluble constituent. The blood sample can be applied to the test device dropwise or with a pipette, or, preferably, the test device can contact a fresh puncture wound and the blood sample is drawn into the test device by capillary action. Appreciably increasing the size of the filter pad substantially increases the time of separation, and also requires a larger blood sample. Furthermore, a relatively large sample of blood can be used to increase the speed of serum transfer to saturate the test pad. In accordance with another feature of the present invention, the amount of whole blood sample contacting the test device does not have to be precisely measured. However, care should be exercised to avoid overloading the filter pad with an excessively large blood sample such that a portion of whole blood contacts the test area of the test device.

As will be discussed more fully hereinafter, the filter pad preferably comprises a carrier matrix incorporating a separating reagent to achieve essentially total removal of the cellular components of whole blood from the plasma or serum. However, the prior art separating reagents, such as the inorganic salts and amino acids disclosed by Fetter in U.S. Patent No. 3,552,925 and 3,552,928, have proven deficient because contaminating ions or molecules are introduced into the serum or plasma, and assayable plasma or serum constituents also are separated from the plasma or serum.

Therefore, in accordance with an important feature of the present invention, a separating reagent, comprising an agglutinizing agent, a coagulating agent or a mixture thereof, is incorporated into the carrier matrix such that the cellular components of the whole blood agglutinate or are trapped as the blood sample chromatographs through the carrier matrix. The agglutinated or trapped cells become fixed, and are collected within the carrier matrix, as the plasma or serum continues advancing through the filter pad to eventually contact and saturate the test pad of the test device.

As will become apparent from the following detailed description of the invention, various lectins or thrombin, used individually or in combination, agglutinate or coagulate the cellular components of whole blood to fix the cells within the carrier matrix, and to allow the undiluted serum or plasma to advance, unaltered and essentially unimpeded, to the test pad of the test device. In addition, the lectins and thrombin of the separating reagent do not promote excessive hemolysis. Therefore, the red blood cells do not rupture and their highly colored components do not interfere with and mask the chromogenic assays.

The lectins are proteins or glycoproteins that are known to agglutinate, or clump, cells and precipitate complex carbohydrates. Lectins are isolated from a wide variety of natural sources, including seeds, plant roots, bark, fungi, bacteria, seaweed, sponges, fish eggs, invertebrate and lower vertebrate body fluids, and mammalian cell membranes. The lectins are often blood group specific, and have been used in blood grouping, polyagglutination studies, and various histochemical studies of normal and pathological conditions. The lectins used in the present invention preferably are not specific to a particular blood group, or the general utility of the process and device of the present invention could be limited. Therefore, among the lectins showing no specificity of blood grouping and that are suitable for use in the present invention are Abrus precatorius (abrin, Jequirty bean), Bauhinia purpurea (camels foot tree), Caragana arborescens (Siberian pea tree), Codium fragile (Green marine algae), Canavalia ensiformis (Con A, Concanavalin A, Jack bean), Glycine max (Soybean), Lathyrus odoratus (Sweet Pea), Lens culinaris (Lentil), Limulus polyphemus (Horseshoe crab, Limulin), Lycopersicon esculentum (Tomato), Maclura pomifera (Osage orange), Mycoplasma gallisepticum, Perseau americana (Avocado), Phaseolus coccineus (Scarlet runner bean), Phaseolus vulgaris (Red Kidney bean), Phytolacca americana (Pokeweed), Pisum sativum (garden pea), Psophocarpus tetragonolobus (winged bean), Ricinus communis (Castor bean), Solanum tuberosum (Potato), Triticum vulgaris (Wheat germ), Vicia faba (fava bean, broad bean), Vigna radiata (Mung bean), Viscum album (European mistletoe), Wisteria floribunda (Japanese wisteria), and other like, non-blood specific lectins.

As will become more apparent hereinafter, the preferred lectins incorporated into the carrier matrix of the filter pad are the lectin from concanavalin A (jack bean), the lectin from solanum tuberosum (potato), the lectin from Triticum vulgaris (wheat germ), the lectin from Bauhinia purpurea (camels foot tree) and the lectin from Phytolacca americana (pokeweed). To achieve the full advantage of the present invention, the carrier matrix of the filter pad is impregnated with the lectin from potato or the lectin from pokeweed.

In addition to, or in place of, the above-described lectins, a coagulating agent also can be used to effect separation of the serum from the cellular components of whole blood. Specifically, the enzyme thrombin, from bovine plasma, has been incorporated into the carrier matrix of the filter pad of the present invention to successfully separate serum from whole blood samples. The bovine thrombin effectively promotes blood clotting such that the red blood cells are removed from the whole blood as the blood permeates through the impregnated carrier matrix. Other thrombins useful according to the method of the present invention include human thrombin, goat thrombin, pig thrombin and sheep thrombin. It also has been found that it is unnecessary to immobilize the thrombin, or the lectins, onto the carrier matrix and that an effective separation of the cellular components from a whole blood sample can be achieved by using the lectins, the thrombin, or a combination thereof.

As previously described, the device of the present invention includes of one or more filter pads, comprising a carrier matrix, or matrices, either untreated or treated with a separating agent, to separate soluble serum or plasma constituents from undiluted whole blood. To achieve the full advantage of the present invention, the filter pad comprises a carrier matrix impregnated with a blood separating reagent, such as a thrombin or a lectin that is not blood group specific. The whole blood sample contacts the filter pad of the test device, whereby the cellular components of the whole blood are separated from the serum or plasma as the blood sample advances through the filter pad. In accordance with an important feature of the present invention, the serum or plasma then advances through the filter pad to contact and saturate a test pad that is in releasable contact with the filter pad. The test pad of the test device of the present invention comprises a suitable substrate material including a suitable indicator reagent composition for the particular assay of interest. After the test pad is saturated with the plasma or serum, the filter pad is separated from the test pad and is detached from the test device, such as by peeling, snapping or tearing the filter pad from the test device. The exposed test pad then is examined, either visually or instrumentally, for a response to a particular analyte of interest.

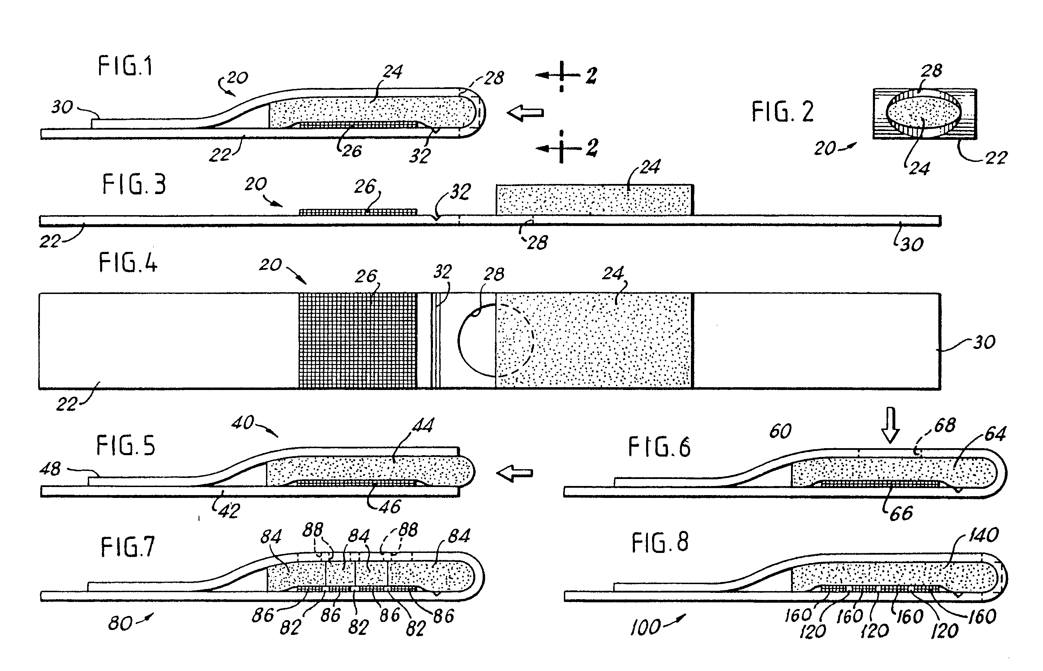

Specifically, the test device of the present invention, and the positioning of the filter pad and the test pad on the test device, is better understood by reference to FIGS. 1 through 9. FIG. 1 shows a test device 20 including a filter pad 24 in releasable contact with a test pad 26. Both the filter pad 24 and the test pad 26 are securely adhered to a support strip or handle 22. The test sample is introduced to the test device 20 in the direction of the arrow at the curved end of the test device 20 through a sample port 28 to first contact and permeate through the filter pad 24. The filter pad 24 separates the cellular components from the whole blood sample, and the plasma or serum advances through the filter pad 24 to contact the test pad 26. After the plasma or serum saturates the test pad 26, an upper free edge 30 of the support strip handle 22 is pulled to separate the filter pad 24 from the test pad 26. By further pulling the upper free edge 30, the support strip or handle 12 is disjoined at a notch 32 to detach the filter pad 24 and the upper free edge 30 from the test device 20 and to expose the test pad 26 for visual or instrumental examination of a response to a particular anaylte of interest. FIG. 2 is an end view of the test device 20 taken in the direction of arrows 2-2 of FIG. 1, and more clearly shows the sample port 28 for introducing the blood sample to the test device 20.

FIG. 3 is a side view and FIG. 4 is a top view of the test device 20 before the support strip or handle 22 is arranged to position the filter pad 24 and the test pad 26 in releasable contact. As will become more apparent hereinafter, in order to facilitate the quantitative determination of plasma or serum constituents, it is preferred that the support strip or handle 22 be manufactured from a hydrophobic, nonabsorbtive material. In addition, the material used in the manufacture of the support strip or handle 22 should be sufficiently pliable to allow the test pad 26 to be disposed in releasable contact with the filter pad 24. To achieve the full advantage of the present invention, for the embodiment illustrated in FIGS. 1 through 4, the filter pad 24 and the test pad 26 preferentially are impregnated with the separating reagent and indicator reagent composition, respectively, before the filter pad 24 and the test pad 26 are adhesively secured to the support strip or handle 22. Alternatively, the separating reagent and the indicator reagent composition can be incorporated into the filter pad 24 and the test pad 26 after the filter pad 24 and the test pad 26 are adhesively secured to the support handle 22, but before the filter pad 24 and the test pad 26 are positioned in releasable contact.

FIGS. 5 and 6 are alternate embodiments of test devices similar to the test device shown in FIG. 1. In FIG. 5, a hydrophobic plastic handle 42 of a test device 40 is adhesively secured to a test pad 46. A filter pad 44 is adhesively secured to a tab 48. The tab 48 is adhesively secured to the handle 42 such that the test pad 46 is in releasable contact with the filter pad 44, and such that the tab 48 has a free, unsecured edge that can be pulled to disconnect the tab 48 and the filter pad 44 from the handle 42 and the test pad 46. The filter pad 44 is positioned to extend beyond the edge of the hydrophobic plastic handle 42 and the edge of the tab 48 to allow the test sample, introduced in the direction of the arrow, to contact the filter pad 44. After the test sample permeates the filter pad 44, and the undiluted and essentially cell-free plasma or serum contacts and saturates the test pad 46, pulling the tab 48 detaches the tab 48 and the filter pad 44 from releasable contact with the test pad 46 and allows examination of the test pad 46 for a response to the analyte of interest.

A test device 60 shown in FIG. 6 is similar to the test device shown in FIG. 1, howevera sample port 68 of the test device 60 is positioned above the filter pad 64, thereby allowing the dropwise addition of the blood sample to the test device 60 in the direction of the arrow. The separation of the cellular components from the plasma or serum is achieved as the whole blood sample advances downward through the filter pad 64. The essentially cell-free plasma or serum then contacts and saturates the test pad 66 for an assay of the particular soluble constituent of interest. The filter pad 64 is separated from the test pad 66 and detached from the test device 60 in an identical manner to separating and detaching the filter pad from the test device illustrated in FIG. 1.

FIGS. 7 and 8 are alternate configurations of a test device similar to the test devices shown in FIG. 6 and FIG. 1. In FIG. 7, a test device 80 includes a plurality of sample ports 88, a plurality of filter pads 84, and a plurality of test pads 86, thereby allowing the single test device 80 to assay a whole blood sample for several soluble plasma or serum constituents. In accordance with the test device shown in FIG. 7, each test pad 86 has incorporated therein a different indicator reagent composition capable of assaying for a particular soluble plasma or serum constituent of interest. In the embodiment illustrated by FIG. 7, each test pad 86 must be sufficiently spaced, or include a barrier 82 between each test pad 86, to avoid the serum or plasma saturating a particular test pad 86 from contacting a second test pad 86, otherwise the different indicator reagent compositions in each test pad 86 will commingle and yield a faulty assay. A test device 100 shown in FIG. 8 is similar to the test device of FIG. 1 and FIG. 7 except the test device 100 of FIG. 8 includes a single filter pad 140 disposed over, and in releasable contact with, all of the test pads 160, that are separated by barriers 120.

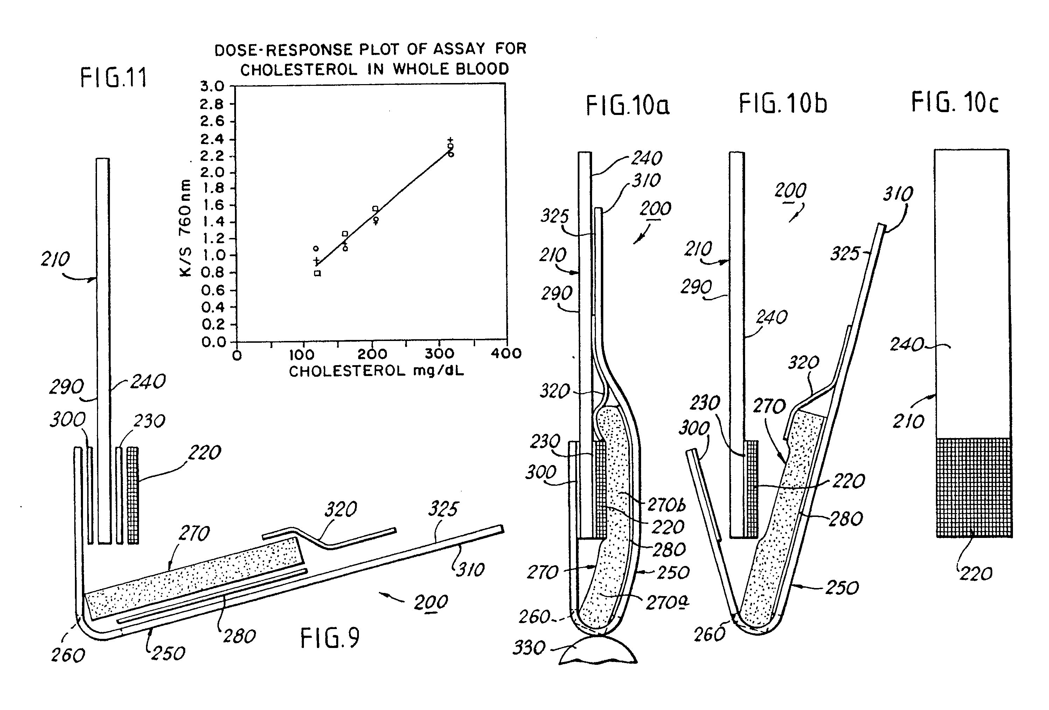

FIG. 9 illustrates the preferred embodiment of the test device of the present invention wherein a test pad 220 of a test device 200 is securely affixed to a top face 240 of a hydrophobic support strip 210 by an adhesive layer 230. Similarly, a filter pad 270 is securely affixed to a detachable envelope strip 250 by an adhesive layer 280. The filter pad 270 is positioned on the detachable envelope strip 250 sufficiently close to a sample port 260 such that contacting the sample port 260 of the test device 200 with a test sample allows the test sample first to contact the filter pad 270. In addition, in the test device 200, the test pad 220 is positioned sufficiently distant from the sample port 260 such that the test sample contacts only the filter pad 270 first and then contacts the test pad 220 after the cellular components of the whole blood sample have been removed by the filter pad 270.

The adhesive composition utilized in the adhesive layer 230 and in the adhesive layer 280 can be any adhesive composition that possesses sufficient tackiness to maintain secure contact between the top face of the hydrophobic support strip 240 and the test pad 220, and between the filter pad 270 and the detachable envelope strip 250, such that the filter pad 270 is removed with the detachable envelope strip 250, and such that the test pad 220 remains attached to the top face 240 of the support strip 210 when the detachable envelope strip 250 is detached from the test device 200. Consequently, the adhesive layer 230 and the adhesive layer 280 should not be adversely affected by contact with serum or plasma or with a whole blood sample, and should not include extractable components that could contaminate the test sample and therefore lead to inaccurate or untrustworthy assay results. Examples of suitable adhesive compositions useful in the adhesive layer 230 and the adhesive layer 280 include, but are not limited to, silicone-based adhesives, rubber-based adhesives and acrylic-based adhesives. Such adhesives are commercially available and are well known to those skilled in the art of designing dry phase test strips.

The detachable envelope strip 250 then is secured to a bottom face 290 of the support strip 210 at a position on the bottom face 290 essentially beneath the test pad 220. A layer of adhesive composition 300 used to secure the detachable envelope strip 250 to the bottom face 290 of the support strip 210 is limited only in that the adhesive composition should provide sufficient adhesive strength to maintain the detachable envelope strip 250 in contact with the bottom face 290 of the support strip 210 and also possess sufficiently low adhesive strength to allow separation of the detachable envelope strip 250 from the bottom face 290 of the support strip 210 when an upper free edge 310 of the detachable envelope strip 250 is pulled.

The top face 240 of the support strip 210 also is adhesively secured to the detachable envelope strip 250 and the filter pad 270 by adhesive layer 320 that is applied to the filter pad 270 and to the bottom face 325 of the upper free edge 310 of the detachable envelope strip 250 such that the adhesive layer 320 does not contact either the test pad 220, the whole blood sample or the plasma or serum. It also should be noted that the adhesive layer 320 may prevent any excess serum or plasma, or any excess whole blood sample, from permeating through the filter pad 270 to contact the top face 240 of the hydrophobic support strip 210. Accordingly, only the test pad 220 is saturated with serum or plasma and the top face 240 of the hydrophobic support strip 210 is free of serum or plasma that could drip and contact the technician or could otherwise interfere with the assay. Consequently, a more accurate and reliable assay results, and the technician is further protected from contacting a potentially infectious blood or other biological sample.

As previously stated, the particular indicator reagent composition for the assay of interest usually is incorporated into the test pad 220 before the test pad 220 is adhesively secured to the top face 240 of the support strip 210. The test pad 220 therefore comprises a substrate material impregnated with an indicator reagent composition suitable for the assay of the soluble plasma or serum constituent of interest. The substrate material of the test pad can include any bibulous or nonbibulous material known to those skilled in the art of designing dry phase diagnostic test strips. Similarly, if the filter pad 270 includes a lectin, a thrombin or a combination thereof, the carrier matrix of the filter pad 270 usually is impregnated with the lectin and/or thrombin before the filter pad 270 is adhesively secured to the detachable envelope strip 250. However, alternatively, the indicator reagent composition can be incorporated into the test pad 220 after the test pad 220 is adhesively secured to the top face of the support strip 210; or the lectin and/or thrombin can be impregnated into the filter pad 270 after the filter pad is adhesively secured to the detachable envelope strip 250. In any event, the indicator reagent composition is incorporated into the test pad 220 and the lectin and/or the thrombin is impregnated into the filter pad 270 before the detachable envelope strip 250 is adhesively secured to the bottom face 290 of the support strip 210.

EXAMPLE 1

IMPREGNATION OF THE CARRIER MATRIX OF THE FILTER PAD

The agglutinizing agent, coagulating agent or combination thereof, such as bovine thrombin, the lectin from solanum tuberosum, the lectin concanavalin A, or the lectin from phytolacca americana, is solubilized in normal, or isotonic, saline solution. A carrier matrix, such as filter paper, like WHATMAN CCP500 filter paper, or a glass fiber matrix, like WHATMAN PD107, available from Whatman Ltd., Maidenshead, Kent, U.K., then is impregnated with the lectin- and/or thrombin-containing saline solution either by immersing the carrier matrix into the saline solution or by spraying the saline solution onto sheets or precut strips of the carrier matrix. The impregnated carrier matrix then is dried at about 50° C. for about 20 to about 40 minutes prior to use.

It is not necessary to immobilize the thrombin or lectin when impregnating the carrier matrix as in Example 1. The simple drying technique is sufficient to maintain the lectin or thrombin in place within the carrier matrix for separation of the cellular components from whole blood. The carrier matrix, after appropriate sizing, e.g., 0.5 cm x 1.0 cm, then can be secured to either a transparent or an opaque, hydrophobic plastic handle such as the support strip or handle 22 in FIG. 1, or the detachable envelope strip 250 in FIG. 9.

Tests to determine the ability of the coagulating agent bovine thrombin and the agglutinizing agents concanavalin A, solanum tuberosum, triticum vulgaris and phytolacca americana to agglutinate or clot blood were run at concentrations ranging from 90 to 900 NIH units/cm$^3$ of thrombin; 140 to 9200 units/cm$^3$ concanavalin A; 4000 to 40,000 units/cm$^3$ solanum tuberosum lectin; 115 to 1150 units/cm$^3$ triticum vulgaris lectin; and 57 to 570 units/cm$^3$ phytolacca americana lectin. In each case, a carrier matrix was impregnated with a separating reagent in accordance with the method of Example 1, and the carrier matrix was securely affixed to a plastic handle or substrate. An untreated pad of filter paper was adhered adjacent to, and in contact with, the impregnated carrier matrix. For each test the amount of plasma or serum separated from whole blood was determined, and the time of separation noted. The whole blood was deposited on the separating reagent-impregnated carrier matrix and allowed to advance, chromatographically, through the impregnated carrier matrix to the untreated filter paper substrate. Table I includes the optimum concentration range found for each separating reagent and the percent of separated plasma or serum tranferred to the untreated pad of filter paper.

## TABLE I

| Separating Reagent | Optimum Concentration Range | Percent of Plasma Observed |
|---|---|---|
| Thrombin | 300-450 NIH units/cm$^3$ | 40% |
| Concanavalin A | 600-920 units/cm$^3$ | 40% |
| Solanum tuberosum | 12000-16000 units/cm$^3$ | 100% |
| Phytolacca americana | 570 units/cm$^3$ | 60% |
| Triticum vulgaris | 1150 units/cm$^3$ | 80% |

Identical tests were performed in the presence of calcium chloride or manganese sulfate because these salts have been proposed as blood clotting accelerators. However, when used in conjunction with the lectin or thrombin separating reagents, and at concentrations of 0.5%, 1.0%, and 3.0% of calcium chloride or manganese sulfate, no improvements in time of the separation or efficiency of the separation was observed. Similarly, to improve the wetting of the carrier matrix, the anionic surfactant, sodium alpha-olefin sulfonate, was impregnated into the carrier matrix, at about 0.1% by weight, in addition to the separating reagent. The wetting of the carrier matrix was improved, therefore reducing the time for plasma or serum separation. However, the amount of separated plasma or serum was not increased. Identical results were observed when a combination of calcium chloride and sodium alpha-olefin sulfonate was included with the thrombin or lectin separating reagent. Accordingly, no appreciable benefits are observed if an inorganic salt or surfactant is included in the separating reagent, plus the inorganic salt or the surfactant have the disadvantage of possibly contaminating the plasma or serum and therefore providing an inaccurate assay.

In accordance with another important feature of the present invention, the filter pad of the test device effectively separated the cellular components of whole blood from the plasma or serum even in the presence of anticoagulants, provided that the filter pad includes a separating reagent comprising a lectin, a thrombin or a combination thereof. Specifically, whole blood containing anticoagulants, such as heparin or ethylenediaminetetraacetic acid (EDTA), is amenable to the separation and testing method and the device of the present invention. Therefore, fresh blood samples can be treated with anticoagulants and then tested by the process and device of the present invention at a later date. In accordance with another important feature of the present invention, hemolysis is essentially eliminated in separations utilizing lectins and was minimal in separations utilizing thrombin. The degree of lysis in the thrombin examples was such that the coloration from the highly colored red blood cells did not materially interfere in any subsequent assays for plasma- or serum-soluble constituents.

In addition to an unexpectedly efficient separation of the red blood cells from the plasma or serum, the process and device of the present invention allow a sufficiently large and assayable amount of plasma or serum to reach the test pad of the test device. Furthermore, the plasma or serum reaches the test pad of the test device in an essentially unaltered form. Generally, the proper amount of serum or plasma has reached the test pad when the test pad is saturated with plasma or serum. This is accomplished either by using a sufficiently large whole blood sample to assure plasma or serum saturation of the test pad, or, preferably, by adjusting the relative sizes of the filter pad and the test pad such that the test pad is saturated with plasma or serum. Generally, the size of the filter pad is directly related to the predetermined blood sample size and, if present, the separating reagent utilized. A precisely measured whole blood sample volume is not necessary. This process allows for an essentially fixed amount of plasma or serum to reach the test pad, and renders a more accurate soluble constituent determination. The variables of blood sample size, filter pad size, test pad size and the amount of indicator reagent composition impregnated into the test area easily can be determined by those skilled in the art after the particular agglutinizer, coagulant or combination thereof is chosen as a separating reagent for incorporation into the filter pad and its separation efficiency determined.

In accordance with another important feature of the present invention, essentially none of the plasma- or serum-soluble constituents of the whole blood sample are separated from the plasma or serum along with the cellular components, nor do intracellular components of the cells contaminate the separated plasma or serum. To demonstrate the utility of the present invention, assays were performed on the essentially clear liquid that permeated the test pad after the cellular components were separated by the filter pad. Surprisingly, the method and device of the present invention separated the plasma or serum from the cellular components with no observable increase in potassium ion or loss of cholesterol, and with essentially no evidence of red blood cells. In addition, the white blood cells also were separated from the whole blood sample. Table II summarizes the results of the assays for potassium ion, cholesterol, red blood cells (erythrocytes) and white blood cells (leukocytes) on the serum or plasma obtained after red blood cell separation. Leukocytes and erythrocytes were assayed using test pads sensitive to leukocyte esterase and the peroxidative activity of hemoglobin, whereas cholesterol sensitive and potassium sensitive test pads were used to assay for those particular constituents. The assays were performed on plasmas or serums obtained from a fresh whole blood sample by incorporating the separating reagents thrombin, the lectin from solanum tuberosum, the lectin from phytolacca americana or the agent used in Fetter U.S. Patent No. 3,552,925, namely sodium sulfate, into a filter pad of the present invention.

## TABLE II

### PLASMA OR SERUM ASSAYS

| Separating Reagent | Leukocytes | Blood | Potassium Ion | Cholesterol |
|---|---|---|---|---|
| Thrombin | Negative | Positive | Positive | Unknown (lysis blocked determination) |
| Solanum tuberosum lectin | Very slight positive | Slight positive | Positive | Positive |
| Phyto-lacca americana lectin | Slightly positive | Negative | Positive | Positive |
| Sodium sulfate | Inconclu-sive | Slight positive | Positive | Negative |

Positive – observable reaction

Negative – no observable reaction

From the data summarized in Table II, it is seen that the salts used in the Fetter method precipitate the high molecular weight constituents of plasma or serum, such as cholesterol, therefore making such constituents unavailable for assay at the test pad of the test device. However, the lectin and thrombin separating reagents do not promote precipitation of high molecular weight plasma or serum constituents. Although thrombin promoted sufficient lysis to block the assay of cholesterol in serum, lysis can be controlled by using normal saline, as opposed to phosphate-buffer saline, when impregnating a carrier matrix with the thrombin separating reagent solution. Hemolysis inhibiting agents also can be incorporated into the carrier matrix used in the filter pad of the present invention. Therefore, high molecular weight components of plasma or serum can be assayed by using any of the lectin or thrombin separating reagents. Also, in certain instances, hemolysis, or blood staining, may be desirable since assays then can be performed on the constituents present within the red blood cells.

In addition to the fast and efficient separation of the serum or plasma from the cellular components of whole blood, and the essentially unimpeded migration of unaltered plasma or serum to the test pad, the method and device of the present invention allow a quantitative assay of a plasma- or serum-soluble constituent without dilution of the whole blood or plasma and without interference from the highly-colored red blood cells. Testing the undiluted serum or plasma both omits a manipulative step and, more importantly, eliminates the possibility of technician error and technician contact with the potentially infectious test sample.

A suitable chromogenic indicator reagent composition is impregnated into the test pad in a sufficient amount to allow an essentially immediate interaction with the freshly separated and undiluted plasma or serum. The extent of the chromogenic reaction, and therefore the quantitative amount of the soluble constituent, then is determined by chromogenic detection techniques, either visual or instrumental, that are well-known in the art. In addition, the cell-contaminated filter pad is detached from the test device to preclude the cellular components from contaminating the plasma or serum, and therefore providing an accurate and trustworthy assay of the serum or plasma. Accordingly, the method and test device demonstrate appreciable advantages over the prior art methods and devices because complicated and expensive multilayered test strips are avoided; the technician does not have to wipe the separated cellular components from the device, thereby avoiding a manipulative step and a potential technician error; and the technician avoids all physical contact with the blood sample.

To more clearly demonstrate the benefits and advantages of the method and test device of the present invention, quantitative assays for total bilirubin, creatinine and cholesterol concentration in a whole blood sample were performed. The assays were performed using a test device as illustrated in FIG. 9, and in accordance with the method illustrated in FIG. 10a-10c. Therefore, in general, to perform an assay for a particular soluble constituent of whole blood, or other biological fluid, as illustrated in FIG. 10a, the sample port 260 of the test device 200 contacts a whole blood sample 330. The whole blood sample 330 can be applied to the sample port 260 of the test device 200, or, preferably, the sample port 260 of the test device 200 is allowed to contact a fresh, small puncture wound to withdraw the blood sample 330 directly from an individual. After the sample port 260 has withdrawn, or contacted, a sufficient amount of blood, it is removed from the wound. The whole blood sample 330 then advances through the filter pad 270, wherein the cellular and particulate components of the whole blood sample 330 are separated, collected and retained in the region of the filter pad 270 designated as 270a. The essentially cell-free plasma or serum of the whole blood sample 330 advances chromatographically through the filter pad 270 to saturate the filter pad 270 in a region adjacent to, and in contact with, the test pad 220, and designated as 270b. The essentially cell-free serum or plasma of the whole blood sample 330 then saturates the test pad 220.

The whole blood sample 330 is allowed an approximately 60 second incubation period after the test device 200 contacts the whole blood sample 330 to permeate the filter pad 270 and effect separation of the cellular components from whole blood sample 330 and to saturate the test pad 220 with serum or plasma. Then, as depicted in FIG. 10b, the upper free edge 310 of the detachable filter envelope 250 is lifted and pulled or peeled to detach the detachable filter envelope 250 from releasable contact with the test pad 220 and to disconnect the detachable filter envelope 250 from the test device 200. The detachable filter envelope 250 then is discarded. In FIG. 10c, after an additional incubation period of approximately 60 seconds to allow the indicator reagent composition incorporated into the test pad 220 to completely interact with the soluble plasma constituent of interest, the exposed test pad 220 is examined, visually or by instrument, for a response to determine the presence or concentration of the soluble plasma constituent of interest. After examining the test pad 220 for a response, the test pad 220 and the hydrophobic support strip 210 are discarded.

Accordingly, an accurate and reliable assay of a small volume of whole blood for a particular soluble plasma constituent is achieved within approximately 2 minutes, without interference by the cellular components of the whole blood. In addition the assay is simple, economical and safe because the manipulative steps required in multilayered test devices, like wiping or rinsing of the test device, and in physical separation methods, like centrifuging, are unnecessary. Furthermore, the device and method are safe because the technician is effectively shielded from any inadvertent contact with the potentially infectious whole blood sample. The entire whole blood sample, both the cellular components in the filter pad and the plasma or serum in the test pad, is retained by the elements of the test device. Therefore, after discarding the elements of the test device, human contact with the test device is essentially precluded. Such a feature is new in the art, wherein prior art methods and devices for testing serum or plasma led to the possibility of human contact with the blood sample, either in the wiping or rinsing of the cellular material from the test device or in blood transfers and dilutions in centrifuging and related physical separation methods.

Accordingly, Examples 2 through 4 more fully and specifically demonstrate assays performed in accordance with the method and test device of the present invention for particular soluble plasma or serum constituents.

## EXAMPLE 2

### DETERMINATION OF TOTAL BILIRUBIN IN PLASMA OR SERUM

An undiluted whole blood sample was assayed for total bilirubin by a test device as illustrated in FIG. 9. Similarly, an undiluted whole blood sample can be assayed for total bilirubin by a test device as illustrated in FIG. 1 and in FIGS. 5 through 8. The test device contacted a whole blood sample, such as a pinprick amount, at the sample port of the device, and the whole blood sample was absorbed by a filter pad comprising a carrier matrix containing a lectin separating reagent. The blood sample chromatographed through the impregnated filter pad, whereby the cellular components of the whole blood were separated from the plasma or serum. The blood sample was of sufficient size such that after chromatographing through the lectin-impregnated filter pad, the amount of serum or plasma in the sample was sufficient to completely wet, or saturate, a test pad that was in releasable contact with the filter pad. After saturation of the test pad by the plasma or serum, the filter pad, contaminated with the cellular components of the whole sample, was detached from the test device by peeling, tearing or snapping off the portion of the test device handle secured to the filter pad. The filter pad was detached from the test pad to expose the test pad for examination of a response, i.e., the chromogenic change, to the total bilirubin content in the serum or plasma. An indicator reagent composition comprising 0.4% w/w 2,4-dichloroaniline, 1.1% w/w sodium nitrite, 57.2% w/w dyphylline, 35.5% w/w buffer, and 5.8% w/w nonreactive ingredients, and previously impregnated into the test pad of the test device, interacted with the bilirubin in the serum or plasma to produce a measurable chromogenic change that correlates quantitatively to the total bilirubin content in the serum or plasma.

The chromogenic change in the test pad was determined visually, such as by a comparison to a standardized color chart, or, alternatively, instrumentally, such as by a reflectance measuring instrument. When using a reflectance photometer, the method provided a response to the concentration of total bilirubin in an undiluted serum or plasma sample without any interference from the cellular components of the whole blood sample. The assay method was based on the van den Bergh reaction, modified to use 2,4-dichloroaniline and a diazo coupling accelerator. The final product, azobilirubin, behaved as an indicator, that is red-purple in color under acid conditions. The concentration of total bilirubin in a sample was quantified from a calibration curve.

A calibration curve was generated internally. Once the calibration curve was generated, each assay required approximately 60 uL (microliter) of whole blood and one device of the present invention. After an incubation period of about 75 seconds, the concentration of total bilirubin in the test sample was determined by measuring the change in reflectance at 560 nanometers (nm) with reference to a calibration curve generated using calibrators. Preferably, the filter pad was separated from the test pad prior to the 75 second incubation period to preclude inadvertent contamination of the plasma or serum by the cellular components of the test sample. Results were obtained in mg/dL or umol/l directly from a reflectance photometer. No calculations are required. The test responded to from 0.4 mg/dL to 7.5 mg/dL (7 umol/l to 130 umol/l) serum or plasma total bilirubin. Serum total bilirubin values of 0.1 mg/dL to 1.2 mg/dL (1.7 umol/l to 20.5 umol/l) have been suggested as the adult normal range.

EXAMPLE 3

DETERMINATION OF CREATININE IN PLASMA OR SERUM

The identical method described in Example 2 was used to quantitatively determine the amount of creatinine in undiluted whole blood samples. However, the indicator reagent composition used in the creatinine assay comprises 43.5% w/w potassium hydroxide, 55.8% w/w 3,5-dinitrobenzoic acid, and 0.6% w/w nonreactive ingredients.

When used with a reflectance photometer, the method provides a direct reading of the concentration of creatinine in the test sample. The assay method is based on the Benedict-Behre reaction wherein creatinine interacts with 3,5-dinitrobenzoic acid in an alkaline medium to form a purple-colored complex. The concentration of creatinine in the sample is quantified from a calibration curve generated using calibrators.

During an incubation and test period of about 30 seconds, the concentration of creatinine in the sample is determined by measuring the rate of change in reflectance at 560 nm with reference to a calibration curve generated using calibrators. The result is displayed digitally by a reflectance photometer. As described above, it is preferred that the filter pad is detached from the test pad as soon as practicable, such as about 60 seconds after contacting the whole blood sample, to eliminate the possibility of inadvertent contamination of the plasma or serum.

A calibration curve is internally generated. Once the calibration curve has been generated, each assay requires approximately 60 uL of whole blood sample and one device of the present invention. By using a reflectance photometer, no calculations are required. The test covers a range of 0 mg/dL to 15 mg/dL (0 umol/l to 1326 umol/l) serum or plasma creatinine. Serum creatinine values of 0.6 mg/dL to 1.2 mg/dL (53 umol/l to 106 umol/l) for males and 0.5 to 1.0 mg/dL (44 to 88 umol/l) for females have been suggested as the normal range. Nevertheless, the range afforded is sufficiently large to test populations having a creatinine concentration several times higher than the normal suggested range.

EXAMPLE 4

DETERMINATION OF CHOLESTEROL IN PLASMA OR SERUM

Undiluted whole blood samples, including a standardized amount of cholesterol, were assayed for cholesterol content by using test devices as illustrated in FIG. 9. Each test device included a test pad comprising a substrate material of either a polyamide, such as nylon, like BIODYNE B, having a 3 u (micron) pore size and available from Pall Corporation, or a polyvinylidenefluoride, like DURAPORE, having a 0.65 u pore size, available from Millipore Corporation, Bedford, MA. The substrate material of the test pad was impregnated with an indicator reagent composition first by immersing the substrate material into an aqueous solution including about 1.5% (w/w) tetramethylbenzidine hydrochloride indicator dye, and about 0.4% (w/w) poly(methyl vinyl ether/maleic anhydride) (GANTREZ AN-139, available from GAF Chemicals Corp., Wayne, NJ.). The impregnated substrate material then was dried in a hot air convection oven at about 50°C for from about 5 minutes to about 10 minutes. The impregnated substrate material then was immersed in a second aqueous solution including:

```
0.2 M Phosphate Buffer (pH 6.0)        66.6% (w/w)
Tetramethyldecynediol ethoxylated
     with 30 moles ethylene oxide -
     (SURFYNOL 485, available from Air
     Products and Chemicals, Inc.,
     Allentown, Pa.)                   1.3% (w/w)
Glycerol                               6.4% (w/w)
Sodium taurocholate                    0.7% (w/w)
Peroxidase                             500 U/mL
Cholesterol Esterase                   500 U/mL
Cholesterol Oxidase                    250 U/mL
Polyvinylpyrrolidione (PVP K-60
     available from GAF Chemicals
     Corp., Wayne, NJ) (45% w/w)       23.8% (w/w)
```

After the second impregnation, the substrate material again was dried in a hot air convention oven at about 50°C for from about 5 minutes to about 10 minutes to provide a test pad to determinethe amount of cholesterol in a liquid test sample.

Each test device also included a filter pad comprising a carrier matrix impregnated with a lectin separating reagent. The carrier matrix, a glass fiber matrix, having a pore size of from about 1 to about 6 u, and available from Whatman Ltd. or Millipore Corporation, was impregnated with an aqueous solution including about 0.05% (w/w) of the lectin from Phytolacca Americana and about 0.05% (w/w) of an ethoxylated octylphenol including about 5 moles of ethylene oxide (TRITON X-45, available from Rohm and Haas Co., Philadelphia, Pa.). After impregnating the glass fiber matrix with the separating reagent, the impregnated glass fiber matrix was scraped with glass rods to remove any excess aqueous solution. After drying the scraped and impregnated glass fiber matrix, the glass fiber matrix was dried in a hot air convection oven at about 50°C for about 30 minutes to provide a filter pad of the present invention.

The test pad and the filter pad then were positioned in a test device as illustrated in FIG. 9 to assay whole blood samples including a known, standardized amount of cholesterol. Each assay required approximately 60 ul of whole blood sample and one test device of the present invention. Test devices as illustrated in FIG. 9 were used to assay whole blood samples including 120, 162, 205 and 317 mg/dL of cholesterol. Assays on samples of each cholesterol concentration were performed in triplicate. The results are tabulated in TABLE III and graphed in FIG. 11 showing a plot the Kubelka-Munk function (K/S) versus cholesterol concentration. The chromogenic cholesterol assay was detected instrumentally from the change of reflectance observed at 760 nm (nanometers).

The assays were performed by first making a small puncture wound in any part of the body. The sample port of the test device then contacted whole blood seeping from the puncture. When the sample port of the test device was filled, the test device was removed from the wound. It was observed that the whole blood sample was absorbed sufficiently into the filter pad such that excess whole blood sample did not remain in a pool on the sample port or on any outside surface of the test device. After a sufficient time, such as about 1 minute, the detachable envelope strip was separated from the test device and discarded. After allowing approximately 1 to 2 additional minutes for generation of a complete response, the exposed test pad then was examined instrumentally in a reflectance measuring instrument, i.e., a GLUCOMETER 3 instrument, available from Miles, Inc., Elkhart, IN. Alternatively, the exposed test pad could have been examined visually, such as by visually comparing the test pad to a standardized color chart. After examining the test pad for a response, the test pad was discarded. The method and test device precluded the technician from contacting any surface containing the potentially infectious whole blood sample.

The color transition resulting from an interaction between the cholesterol-containing serum or plasma of the whole blood sample with the indicator reagent composition incorporated into the test pad was examined by reflectance photometry at 760 nm. In general, comparison of the color transition resulting from contacting a test pad with a blood sample of unknown cholesterol concentration to the color transition resulting from a test pad contacting a standardized cholesterol concentration gives a quantitative assay for the cholesterol concentration in the blood sample. Therefore, a dose response plot was graphed (FIG. 11) to demonstrate that the intensity of the color transition of the assay varied in direct proportion to the cholesterol concentration of the test sample.

Individual assay results were determined by taking a reflectance measurement with a reflectance photometer, approximately 2 to 3 minutes after the whole blood sample contacted the test device, at a wavelength of 760 nm (nanometers). The reflectance, as taken from the reflectance scale of zero to one, was incorporated into the Kubelka-Munk function:

$$K/S = (I-R)^2/2R,$$

wherein K is the absorption coefficient, S is the scattering coefficient and R is reflectance. The K/S values were plotted against the cholesterol concentration of the test sample. Generally, it can be stated that as reflectance decreases, the K/S value increases.

Therefore, FIG. 11 shows the K/S values versus the cholesterol concentration of the standardized whole blood samples. Each test was run in triplicate, using two instruments. The plotted K/S values are the average K/S values for three replicate trials. The reflectance data and the standard deviation over the replicate trials is tabulated in TABLE III. The linear dose response plot illustrated in FIG. 11 demonstrates the accuracy of the assay procedure for cholesterol, and especially demonstrates that the filter pad of the test device effectively eliminated the interferences attributed to the cellular components of the whole blood samples while allowing the analyte of interest to chromograph through the filter pad to saturate the test pad. The data and dose response plot also demonstrate that the test device and method of the present invention permit the effective removal of assay interferants followed by fast, safe, economical and accurate detection of the assay response by currently available instrumental, or alternatively visual, detection techniques.

## TABLE III

### WHOLE BLOOD ASSAY FOR CHOLESTEROL

|  | CHOLESTEROL CONCENTRA-TION | ASSAY 1 | ASSAY 2 | ASSAY 3 | AVG | STANDARD DEVIATION |
|---|---|---|---|---|---|---|
| %R | 120 | 30.6 | 27.8 | 26.0 | 28.1 | 1.89 |
| | 162 | 23.5 | 25.1 | 25.9 | 24.8 | 1.00 |
| | 205 | 20.6 | 22.2 | 21.8 | 21.5 | 0.68 |
| | 317 | 15.6 | 15.3 | 16.2 | 15.7 | 0.37 |
| K/S | 120 | 0.787 | 0.938 | 1.053 | 0.926 | 0.109 |
| | 162 | 1.245 | 1.118 | 1.060 | 1.141 | 0.077 |
| | 205 | 1.530 | 1.363 | 1.403 | 1.432 | 0.071 |
| | 317 | 2.283 | 2.345 | 2.167 | 2.265 | 0.073 |

With the appropriate chemistry, assays on undiluted plasma or serum, utilizing known chromogenic reactions and a reflectance spectrophotometer, also can be performed for uric acid, potassium ion, glucose, galactose, urea, phenylalanine, triglycerides, various enzymes and other soluble constituents of whole blood or other biological samples. See, for example, U.K. Patent No. 2,014,155; U.S. Patent No. 4,186,251; U.S. Patent No. 4,057,394; and related patents, hereby incorporated by reference. In general, because the contaminated filter pad is completely removed from the test device to expose a simple test pad, less bulky, less complicated, and more economical instrumentation devices can be used to detect the chromogenic change in the test pad. Performing an assay with a complicated, prior art multilayered test strip requires the use of more expensive instrumentation, and increases the possibility of an inaccurate assay because manipulative steps, such as wiping the cellular components from a semipermeable membrane, are necessary. In addition, the chromogenic change can be determined by simple visual techniques, such as standard color chart comparisons, because the technician examines a simple test pad saturated only by the serum or plasma, as opposed to examining a test pad also including the separated cellular components of the whole blood sample. Accordingly, the test pad of the test device of the present invention can be examined, easily and accurately, by relatively untrained and nontechnical laboratory personnel.

### EXAMPLE 5

To further show that a test device of the present invention can include a filter pad absent a separating reagent, a 0.5 centimeter square pad of untreated glass fiber matrix, such as a PD-25 glass fiber matrix, available from Millipore Corporation, was used as the filter pad in a test device of the present invention as illustrated in FIG. 9. The untreated glass fiber matrix pad was approximately 1 mm thick. The test pad was a blank pad of filter paper. The whole blood sample contacted the untreated glass fiber matrix filter pad, advanced through the filter pad and ultimately saturated the test pad. After detaching the filter pad from the test pad, a saturated and essentially colorless test pad was exposed, therefore showing that the untreated filter pad effectively separated the cellular components of the whole blood sample and precluded contamination of the test pad of the test device.

It will be understood that the present disclosure has been made only by way of preferred embodiment and that numerous changes in details of construction, combination, and arrangement of parts can be resorted to without departing from the spirit and scope of the invention as hereunder claimed.

### Claims

1. An analyte test device that separates cellular and particulate components from a liquid test sample and that determines the presence or concentration of a soluble component of the liquid test sample comprising:

    a support strip;

    a test pad secured to the support strip, the test pad comprising a substrate material incorporating an indicator reagent composition that undergoes a detectable change in response to the soluble component of the liquid test sample when in contact with the liquid test sample;

a filter pad of sufficient size to separate the cellular and particulate components from the liquid test sample, the filter pad being disposed in releasable contact with the test pad and so positioned that the liquid test sample first contacts the filter pad; and

a removable securing means secured to the filter pad and to the support strip to dispose the filter pad in releasable contact with the test pad, whereby separating the securing means from the support strip disconnects the filter pad from the test pad to expose the test pad for examination of the detectable change in response to the soluble component of the test sample.

2. The analyte test device of claim 1 wherein the biological sample is whole blood.

3. The analyte test device of claim 1 wherein the filter pad comprises a carrier matrix selected from the group consisting of silica gel, alumina, diatomaceous earth, filter paper, chromatographic paper, glass fiber, cellulose acetate, polyvinyl chloride, polyacrylamide, polyacrylate, polyurethane, crosslinked dextran, polyethylene, polypropylene, polyvinylidene fluoride, polysulfone and combinations thereof.

4. The analyte test device of claim 1 wherein the filter pad comprises a carrier matrix having a pore size in the range of from about 0.1 micron to about 50 microns.

5. The analyte test device of claim 1 wherein the filter pad has a thickness of at least about 1 mm.

6. A device for separating plasma or serum from a biological fluid and for detecting a soluble constituent of the plasma or serum comprising a filter pad to remove the cellular and particulate components of the biological sample upon contact therewith, and a test pad containing a suitable indicator reagent composition in a sufficient quantity for interaction with a soluble constituent of the plasma or serum to produce a detectable change in the test pad upon contact with the serum or plasma, the filter pad being so positioned that the biological fluid first contacts the filter pad to remove the cellular and particulate components of the biological fluid and the test pad being disposed in releasable contact with the filter pad and so positioned that the interaction between the soluble constituent of the plasma or serum and the indicator reagent composition is detected without substantial interference from the cellular and particulate components separated by the filter pad after the filter pad is disconnected from releasable contact with the test pad.

7. A device for separating plasma or serum from an undiluted whole blood sample comprising a filter pad to separate the cellular and particulate components from the sample in the area of the filter pad while allowing a quantity of the plasma or serum portion of the whole blood sample to flow completely through the filter pad in undiluted form separate from the cellular and particulate components of the whole blood sample into a test pad, the test pad disposed in releasable contact with the filter pad and including a suitable indicator reagent composition to assay for a particular soluble constituent of the serum or plasma, whereby disconnecting the filter pad from the test pad exposes the test pad for examination of a response to the particular soluble constituent of the serum or plasma.

8. A method of separating plasma or serum from an undiluted whole blood sample comprising contacting the undiluted whole blood sample with a filter pad of a device to separate and retain the cellular and particulate components of the undiluted whole blood sample in the filter pad while allowing a quantity of the plasma or serum portion of the undiluted whole blood sample to flow through the filter pad of the device into a hydrophilic pad of the device that is in releasable contact with the filter pad, then disconnecting the filter pad from the hydrophilic pad to expose the plasma or serum-containing hydrophilic pad.

9. A method of separating plasma or serum from a whole blood sample and testing the plasma or serum for a predetermined soluble constituent comprising contacting the whole blood with a filter pad to separate and retain the cellular and particulate components from the whole blood thereby separating the plasma or serum from the cellular components of the whole blood; allowing the separated plasma or serum to contact a test pad containing a suitable testing reagent for the soluble constituent of the plasma or serum, the test pad being in releasable contact with the filter pad for contact of the separated serum or plasma with the test pad to produce an interaction between the predetermined soluble constituent and the testing reagent of the test pad resulting in a detectable change in the test pad; detaching the filter pad from releasable contact with the test pad to expose the test pad; and detecting the detectable change in the test pad.

10. A method of determining the presence or concentration of a specific soluble constituent in a biological fluid comprising:

(a) contacting a test device with the biological fluid, the test device comprising:

a support strip;

a reagent test pad secured to the support strip, the reagent test pad comprising a substrate material incorporating an indicator reagent composition that undergoes a detectable change in response to the specific soluble constituent of the biological fluid when in contact with the biological fluid;

a filter pad capable of separating cellular and particulate components from the biological fluid, the filter pad being disposed in releasable contact with the reagent test pad and so positioned that the biological sample first contacts the filter pad; and

a removable securing means secured to the filter pad and to the support strip to dispose the filter pad in releasable contact with the reagent test pad, whereby separating the securing means from the support strip disconnects the filter pad from the reagent test pad to expose the reagent test pad for examination of the detectable change in response to the specific soluble constituent of the biological sample;

(b) allowing a sufficient time for the biological sample to permeate the filter pad to effect separation of the cellular and particulate components of the biological sample and for a liquid portion of the biological fluid to contact the reagent test pad;

(c) disconnecting the filter pad including the cellular and particulate components of the biological sample from the reagent test pad including the liquid portion of the biological fluid and detaching the filter pad from the test device to expose the reagent test pad; and

(d) determining the presence or concentration of the specific soluble constituent from the detectable change of the indicator reagent composition in response to the specific soluble constituent in the biological fluid.

FIG.1

FIG.2

FIG.3

FIG.4

FIG.5

FIG.6

FIG.7

FIG.8

EP 0 436 897 A2

FIG. 10c

220
240
210

FIG.10b
200
310
325
320
270
230
300
220
280
250
260
240
210
290

FIG.10a
240
310
200
325
210
290
320
230
300
270b
220
280
250
270a
270
260
330

FIG.9
325
320
200
310
270
280
250
260
210
290
300
230
240
220

FIG.11

DOSE-RESPONSE PLOT OF ASSAY FOR CHOLESTEROL IN WHOLE BLOOD

K/S 760 nm

3.0
2.8
2.6
2.4
2.2
2.0
1.8
1.6
1.4
1.2
1.0
0.8
0.6
0.4
0.2
0.0

0    100    200    300    400

CHOLESTEROL mg/dL